# DEMANDE DE BREVET EUROPEEN

(11) **EP 3 403 633 A1**
(43) Date de publication de la demande: **21.11.2018**
(21) Numéro de dépôt: 18175650.3
(22) Date de dépôt: 11.12.2015
(51) Int. Cl.: A61F 13/532, A61F 13/537, A61F 13/00

(54) **ARTICLE TEXTILE MASSANT**

(30) Priorité: 11.12.2014 WO PCT/EP2014/077468
(62) Demande divisionnaire de: 15199691.5
(71) Demandeur: SKIN'UP, 37140 Restigne (FR)
(72) Inventeur: BEAUGE-DUGUET, Sophie, 37140 Restigne (FR)
(74) Mandataire: Tripoz, Inès

(57) **Abrégé**

La présente invention a trait à la mise en oeuvre de massages cutanés superficiels.
Il s'agit d'un textile destiné à ête appliqué sur la peau, constitué de fibres, fils ou filaments tissés ou tricotés comprenant :
- au moins une fibre, fil et/ou filament ayant la capacité d'émettre des radiations dans des longueurs d'ondes infrarouge lointain, de préférence entre 4 µm et 1000 µm, ou encore entre 5 µm et 20 µm,
caractérisé en ce que au moins une partie d'une face destinée à être en contact avec la peau présente au moins un relief.

## Description

La présente invention a trait à la mise en oeuvre de massages cutanés superficiels.

On connait l'efficacité des massages notamment pour activer la microcirculation périphérique et leur utilisation pour potentialiser les effets de principes actifs qui sont appliqués sur la peau.

On connait divers textiles et notamment ceux décrits dans les documents ci-dessous cités qui par leur application sur la peau et/ou leur port permettent d'obtenir des effets par exemple sur la circulation et ou d'améliorer l'efficacité de principes actifs.

Ces effets sont obtenus par incorporation de charges minérales dans les textiles qui permettent d'obtenir des filaments polymères qui présentent une capacité d'émission ou d'absorption de radiations infrarouges dans une plage de longueur d'onde située entre 2 µm et 20 µm et conduisent à une élévation de la température de la peau par un effet de réverbération.

On connait par exemple du brevet FR 2 999 613 un matériau textile imprégné de principes actifs encapsulés, dont la libération, par exemple dans la peau, est améliorée par des fibres incorporant des charges minérales.

La demande de brevet WO2012/156805 décrit des fils, fibres ou filaments polymères incorporant une charge céramique pour articles textiles et dispositifs médicaux permettant d'améliorer la cicatrisation de la peau à travers la stimulation du fonctionnement des kératinocytes et des fibroplastes, notamment en activant la synthèse du collagène à la surface de la peau lésée.

Le brevet FR 2 967 573 décrit un kit cosmétique comprenant une formulation topique comprenant un actif cosmétique et un textile constitué par des fibres polymères incorporant des charges minérales. Les effets obtenus sont essentiellement dus à la présence des charges minérales, au rayonnement infrarouge et à l'élévation de la température de la peau.

La demande de brevet FR 2 865 905 décrit un vêtement de sport composé par une enveloppe tricotée élastique adaptée à la contention comportant une structure saillante en relief. Des substances actives peuvent être incorporées selon la technique de microencapsulation, dans un but cosmétique ou hygiénique.

On connait des demandes de brevet JP 2002/363821 ou CN 1139164, des textiles incorporants des matériaux capables d'émettre un rayonnement infrarouge lointain. On trouve des procédés de fabrication de structures fibreuses incorporant des charges minérales dans le brevet US 4,968,531.

Il est également connu de WO 2011/114025 ou de EP 0 347 197 des matériaux textiles comprenant une matrice polymère dans laquelle sont noyées des particules d'un matériau minéral susceptible d'émettre un rayonnement infrarouge lointain. L'étoffe ou le matériau textile est alors enduit dudit matériau composite.

Cependant l'idéal serait de pouvoir apporter un massage localement pour activer la microcirculation périphérique même en l'absence de l'application de principes actifs sur des zones choisies et déterminées du corps et également de pouvoir encore augmenter l'efficacité et surtout de potentialiser les effets sur certaines zones.

En effet par exemple dans un traitement de la cellulite celle-ci est très localisée et il est nécessaire sur certaines zones de renforcer l'efficacité.

Le problème est résolu par la présente invention qui consiste en un textile comprenant au moins une fibre, fil et/ou filament ayant la capacité d'émettre des radiations dans des longueurs d'ondes infrarouge lointain, de préférence entre 4 µm et 1000 µm, et encore entre 5 µm et 20 µm et en ce que au moins une partie d'une face destinée à être en contact avec la peau présente au moins un relief.

Grâce au tricotage ou au tissage les zones présentant un relief vont pouvoir être localisées sur des zones choisies pour appliquer le massage où un effet amélioré est attendu.

Dans un mode de réalisation, le tricotage est dit « tricotage intégral » et le textile selon l'invention ne présente pas de couture (seamless).

Dans un mode de réalisation, le textile est tricoté par la méthode dite de tricotage électronique circulaire (circular electronic knitting) à l'aide de machines bien connues de l'homme du métier telles que « circular electronic knitting machines seamless » du fabricant SANTONI.

On entend par fibre, fil et/ou filament ayant la capacité d'émettre des radiations dans des longueurs d'ondes infrarouge lointain, de préférence entre 4 µm et 1000 µm, et encore entre 5 µm et 20 µm, une fibre, fil et/ou filament qui comprend au moins une charge minérale.

Dans un mode de réalisation, la charge minérale est une particule de céramique qui est incorporée dans la masse polymérique, elle peut également être incorporée, par exemple avant réticulation.

Dans un mode de réalisation la au moins une charge minérale est incorporée pendant la polymérisation. Elle peut également être incorporée lors d'une fusion de la masse polymérique, par exemple préalablement au filage.

Les particules de céramique ont la propriété d'émettre et de réfléchir des rayons infrarouge lointain. Cette émission de rayonnement infrarouge est consécutive à l'absorption de chaleur par les particules de céramique. Lorsque le textile est porté sur la peau, les particules de céramique absorbent l'énergie émise par le corps et la restituent dans les longueurs d'ondes de l'infrarouge lointain en provoquant une légère augmentation de la température corporelle.

Cette augmentation de la température corporelle accélère notamment le flux sanguin cutané et la circulation périphérique.

Ces effets sont notamment décrits dans le brevet FR 2 999 613 au nom de la demanderesse.

Dans un mode de réalisation, l'augmentation de la température cutanée, lorsque le textile selon l'invention est porté sur la peau, est supérieure à 1%, de préférence supérieure à 2%, par rapport à un témoin portant un textile n' ayant pas la capacité d'émettre des radiations dans des longueurs d'ondes infrarouge lointain.

Dans un mode de réalisation, l'augmentation de la température cutanée lorsque le textile selon l'invention est porté sur la peau est supérieure à 1%, de préférence supérieure à 2% ou encore supérieure à 4%, par rapport à un témoin ne portant pas des textiles sur la peau.

La Thermothérapie à Rayonnement Infrarouge Lointain, est reconnue comme pouvant stimuler le métabolisme cellulaire. On reconnait certains bénéfices à la thermothérapie tels que des effets de détoxification, anti-inflammatoire, augmentation de l'irrigation sanguine, amélioration des fonctions immunitaires. Le textile selon l'invention permet ainsi d'obtenir ces bénéfices liés à la thermothérapie.

Dans un mode de réalisation, le textile (1) selon l'invention est caractérisé en ce que le au moins une fibre, fil et/ou filament ayant la capacité d'émettre des radiations représente 10 à 50% en masse des fibres, fils et/ou filaments du textile.

Dans un mode de réalisation, le au moins une fibre, fil et/ou filament ayant la capacité d'émettre des radiations est dans la face (6) destinée à être au contact de la peau.

Dans un mode de réalisation, le au moins une fibre, fil et/ou filament ayant la capacité d'émettre des radiations est dans le relief de la au moins une partie d'une face destinée à être en contact avec la peau présente au moins un relief.

Dans un mode de réalisation préféré, le au moins une fibre, fil et/ou filament ayant la capacité d'émettre des radiations est dans le relief (21, 22, 23, 24, 25, 26).

Le pourcentage en masse des fibres, fils et/ou filaments dans lesquels a été dispersée la au moins une particule de céramique par rapport à la masse totale de l'ensemble des fibres, fils et/ou filaments que comporte le textile est de préférence compris entre 10 et 50%. Lorsque le au moins une fibre, fil et/ou filament ayant la capacité d'émettre des radiations est localisé dans le relief, un pourcentage en masse des fibres, fils et/ou filaments de 10 % permet d'obtenir un résultat équivalent à un pourcentage en masse des fibres, fils et/ou filaments de 40 % répartis de manière uniforme dans le textile.

De manière avantageuse, la quantité de la au moins une particule de céramique représente de 0,5% à 50%, de préférence de 0,5 à 15%, et encore plus préférentiellement de 0,5 à 1,5% par rapport à la masse totale des fibres, fils et/ou filaments dans lesquels la au moins une particule de céramique a été dispersée.

On entend par particules de céramiques, des particules qui ont été obtenues par transformation d'un mélange d'argile, de matière minérale, de terres rares et de métaux précieux, porté à très haute température, de l'ordre de 1000 °C ou plus. En d'autres termes, il s'agit de silicates, dont l'arrangement dépend des conditions physico-chimiques de leur formation.

La au moins une particule de céramique comprend avantageusement au moins un oxyde choisi dans le groupe constitué par Al₂O₃, Fe₂O₃, TiO₂, Cr₂O₃, SiO₂, MgO, ZrO₂, MnO₂, CO₂O₃, Y₂O₃, pris seul ou en mélanges de ceux-ci. Dans un mode de réalisation préféré, il s'agit de MnO₂ ou Co₂O₃ ou encore d'un mélange de ces deux oxydes.

Dans un mode de réalisation préféré, la au moins une particule de céramique comprend au moins 10% en masse de MnO₂.

Une composition préférée de la particule de céramique est la suivante en pourcentage massique :
- TiO₂ : 30 à 40 %
- SiO₂ : 10 à 20 %
- MnO₂ : 10 à 20 %
- Al₂O₃ : 10 à 20 %
- Fe₂O₃ : 5 à 10 %
- MgO : 1 à 5 %
- Co₂O₃, ZrO₂, Cr₂O₃, Y₂O₃ inférieur ou égal à 5% chacun.

Le au moins fibre, fil et/ou filament ayant la capacité d'émettre des radiations dans des longueurs d'ondes infrarouge lointain et comprenant une charge minérale, est caractérisé en ce qu'il est constitué d'au moins un polymère ou mélange de polymères.

Les fibres, fils et/ou filaments n'ayant pas la capacité d'émettre des radiations dans des longueurs d'ondes infrarouge lointain peuvent être également choisis parmi les fibres, fils et/ou filaments constitués d'au moins un polymère ou mélange de polymères, identiques ou différents de ceux de le au moins fibre, fil et/ou filament ayant la capacité d'émettre des radiations dans des longueurs d'ondes infrarouge lointain.

Le choix de la composition des fibres du textile selon l'invention va dépendre de l'utilisation et de l'objectif recherché. Certaines fibres seront utilisées pour leurs propriétés élastiques, d'autres pour leur propriétés antitranspirantes ou imperméables.

Des mélanges de différentes fibres, filaments et/ou fils d'origine naturelle et/ou synthétique peuvent également être utilisés.

Les fibres, fils et/ou filaments peuvent comprendre au moins un polymère ou bien un mélange de polymères. Ledit polymère est avantageusement choisi dans le groupe constitué par les polyamides, les polyuréthanes, les acryliques, les chlorures de polyvinyle (PVC), les copolymères polystyrène-butadiène (SBS), le latex naturel ou artificiel (polyisoprène), les polyesters, les polytétrafluoroéthylènes (PTFE).

Dans un mode de réalisation, le polymère est choisi parmi les polyamides.

Dans un mode de réalisation, le au moins fibre, fil et/ou filament ayant la capacité d'émettre des radiations dans des longueurs d'ondes infrarouge lointain est constitué d'au moins 50% de polyamide.

Dans un mode de réalisation, tous les fibres, fils et/ou filaments sont constitués d'au moins 50% de polyamide.

Dans un mode de réalisation, le textile selon l'invention comprend en outre au moins un principe actif micro-encapsulé.

Le au moins un principe actif est choisi parmi les principes actifs pharmaceutiques ou cosmétiques.

En fonction des zones, des applications et des effets recherchés, la quantité de principes actifs peut représenter 1% à 15% en masse du textile selon l'invention, de préférence de 1 à 10%, ou encore de 1 à 5%.

Le pourcentage n'est pas limité à ces valeurs et peut être adapté en fonction de la posologie recommandée pour ledit principe actif.

On peut également trouver dans le commerce des fibres, fils et/ou filaments pouvant être utilisés pour la fabrication du textile selon l'invention. De manière non limitative, on peut utiliser des fibres, fils ou filaments des fabricants NILIT (sous le nom Innergy®) ou NYLSTAR ou SOLVAY (sous le nom Emana®), en particulier des fibres polyamides (nylon) 6.6 avec technologie infrarouge lointain intégrée.

Les principes actifs cosmétiques peuvent être choisis parmi les principes actifs amincissants, comme par exemple des extraits végétaux ou des substances naturelles, en particulier, la caféine, les extraits de vigne, les extraits d'algues, rafraîchissants, comme par exemple le menthol ou hydratants, comme par exemple l'aloe vera, le beurre de mangue, beurre de karité, arnica, camphre.

Dans un mode de réalisation, un principe actif est le *fucus vesiculosus,* une algue brune connue pour son action liporéductrice, anti-inflammatoire et antioxydante.

Les principes actifs peuvent être lipophiles ou hydrophiles.

On citera parmi les principes actifs lipophiles : les vitamines liposolubles, ainsi que leurs dérivés, comme les rétinoïdes, par exemple le rétinol, la rétinaldéhyde, l'acide rétinoïque, les caroténoïdes, par exemple le tocophérol et ses dérivés ; les polyphénols, par exemple les flavonoïdes, par exemples les isoflavonoïdes, la quercétine, les stylbènes, par exemple le resvératrol, les catéchines, par exemple l'épicatéchine-3-gallate, épigallocatéchine-3-gallate ; les composants de parfumerie comme la vanilline, l'indole ou le menthol, ou plus généralement les huiles essentielles telles que les huiles essentielles d'agrumes, de lavande, de menthe ; les principes actifs pharmaceutiques liposolubles, par exemples la fluvastatine, le ketoprofène, le vérapamil, l'atenolol, la griseofulvine, la ranitidine ; des matières grasses végétales (comme l'huile d'argan, de tournesol) ou le beurre de karité.

Les principes actifs hydrophiles peuvent être choisis parmi les aminoglucosides (gentamicine), les antibiotiques ((3-lactam, sulbenicilline, céfotiame, cefmenoxime), les hormones peptidiques (TRH, leuprolide, insuline), les agents anti-allergiques, antimycotiques, cytostatiques, les sels minéraux (calcium, chlore, magnésium, phosphore, potassium, sodium, soufre), les oligo-éléments (aluminium, brome, cuivre, cobalt, fer, fluor, manganèse, molybdène, iode, sélénium, silicium, vanadium, zinc), les acides aminés (alanine, arginine, asparagine, acide aspartique, cystéine, acide glutamique, glutamine, glycine, histidine, isoleucine, leucine, lysine, méthionine, phénylalanine, proline, sérine, thréonine, tryptophane, tyrosine, valine), les peptides, les protéines, les vitamines hydrosolubles, les polyols, les arômes.

La micro-encapsulation des principes actifs peut consister en un enrobage de particules solides ou de gouttelettes de liquides ou de bulles de gaz par une enveloppe solide : cela regroupe l'ensemble des procédés conduisant à l'obtention de microcapsules individualisées constituées d'un matériau enrobant et une matière active.

Les microcapsules présentent une taille comprise entre environ 1 µm et 1 mm et contiennent typiquement entre 5 et 90 % en masse de principe actif.

Les matériaux enrobants sont des polymères d'origine naturelle ou synthétique, ou des lipides.

Les microcapsules sont généralement sphériques et creuses.

Dans le cadre de la présente invention, les microcapsules renfermant le ou les principes actifs peuvent consister en des microcapsules à base de résine aminoplaste.

Les aminoplastes sont des polymères thermodurcissables. On distingue deux types principaux, les résines urée-formaldéhyde et les résines mélamine-formaldéhyde. Ils sont obtenus par une réaction de polycondensation à partir du comonomère formaldéhyde, le second comonomère portant des groupes amino.

La micro-encapsulation est réalisée en polymérisation *in situ,* à savoir qu'une formule cosmétique (c'est-à-dire des principes actifs) non soluble dans l'eau (lipophile) et une solution aqueuse contenant un polymère hydrosoluble sont mélangées pour former une émulsion.

Par modification du pH et de la température, il se forme un polymère insoluble qui va migrer sur l'huile cosmétique pour former une paroi et va enrober les constituants liposolubles.

Les microcapsules obtenues sont étanches, la libération des principes actifs se fait par rupture.

Dans un autre mode de réalisation de l'invention, les microcapsules sont obtenues à partir de polymères biodégradables (naturels ou de synthèse) tels que la cellulose et ses dérivés, les polysaccharides, le chitosane, la gélatine, les polyols.

Les microcapsules à base de gélatine sont obtenues par coacervation. La réaction se fait par addition d'une gélatine en solution à 50°C à pH neutre au(x) principe(s) actif(s). Il se produit une gélification, puis un durcissement des parois des microcapsules par acidification puis par diminution de la température.

Dans le cadre de la présente invention, les microcapsules peuvent également être à base de silicone. La micro-encapsulation est réalisée par polycondensation à l'interface. Une formule cosmétique non soluble dans l'eau (lipophile) contenant un monomère A liposoluble et une solution aqueuse avec un monomère B hydrosoluble sont mélangés pour former une émulsion. Par modification du pH, les monomères vont migrer à l'interface entre huile et eau et condenser en polymère A+B pour former la paroi de la microcapsule. Les microcapsules obtenues sont étanches ou semi-poreuses, la libération des actifs se fait par rupture et par diffusion (porosité).

Dans un mode de réalisation, les micro-capsules ont une taille comprise entre 1 µm et 1 mm.

Les microcapsules peuvent être fixées au textile par un liant.

Cette fixation peut être réalisée par épuisement qui consiste en une immersion du textile dans un bain contenant les microcapsules, un liant et au moins un tensioactif. La migration des microcapsules du bain vers l'interface textile/eau est obtenue par variation du pH, de la force ionique et de la température.

Elle peut être réalisée par une enduction sur le textile, de préférence sur la face destinée à être en contact avec la peau, d'un mélange contenant les microcapsules, une résine acrylique, vinylique ou pâte de polyuréthane, des additifs et tensioactifs. L'enduction est classiquement réalisée au moyen d'une raclette ou d'une calandre à chaud, elle est de ce fait réservée aux principes actifs et microcapsules qui résistent à la chaleur.

Elle peut être réalisée par foulardage qui est une imprégnation du textile qui est obtenu par un trempage de manière continue, suivi d'un calandrage, le bain comprenant un mélange de microcapsules et de liants spécifiques de types acrylique, styrène-butadiène, vinyl, polyuréthane.
Losque le textile (1) selon l'invention comprend des microcapsules lors de l'application sur la peau, par exemple lors du port d'un vêtement ou d'une bande constituée en textile selon l'invention, la longueur d'onde du rayonnement infrarouge lointain correspondant à celle émise majoritairement par le corps humain (10-14 µm) un phénomène de résonance est créé provoquant une vibration et un frottement des microcapsules comprenant les principes actifs.

De plus, l'augmentation de la température amplifie le processus de rupture des microcapsules renfermant des principes actifs évoqués ci-dessus et la diffusion des principes actifs est ainsi potentialisée.

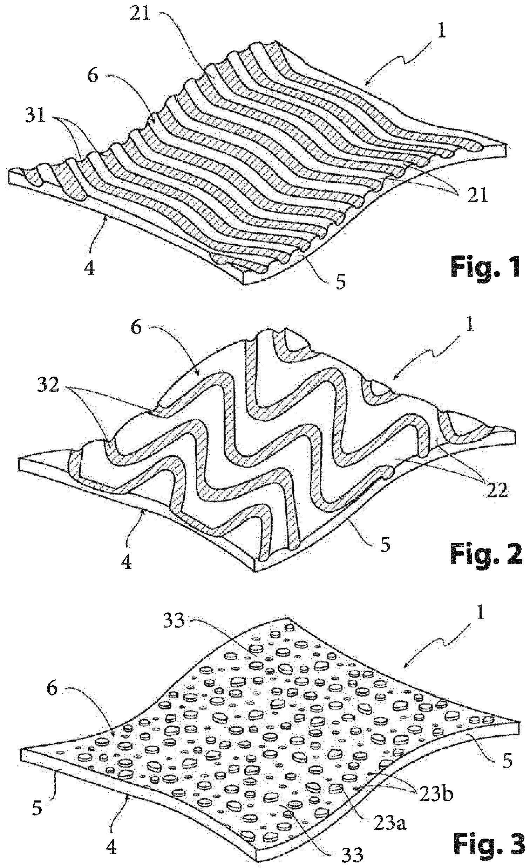

La figure 1 représente un textile (1) selon l'invention, ayant une face destinée à être en contact avec la peau (6) présentant des reliefs (21) en forme de vagues, espacés par des zones sans relief (31) et ayant une face sans relief (4). De tels reliefs (21) en forme de vagues permettent un effet veinotonique (activation du flux sanguin) et reproduisent les effets d'un massage palper-rouler.

La figure 2 représente un textile (1) selon l'invention, ayant une face destinée à être en contact avec la peau (6) présentant des reliefs (22) en forme de vagues, espacés par des zones sans relief (32) et ayant une face sans relief (4). De tels reliefs (22) en forme de vagues permettent un effet veinotonique (activation du flux sanguin) et reproduisent les effets d'un massage palper-rouler.

La figure 3 représente un textile (1) selon l'invention, ayant une face destinée à être en contact avec la peau (6) présentant des reliefs (23a et 23b) en forme de picots, espacés par des zones sans relief (33) et ayant une face sans relief (4). De tels reliefs (23a et 23b) en forme de picots permettent de simuler des points d'acupuncture et peuvent procurer une aide contre des problèmes de contraction musculaire.

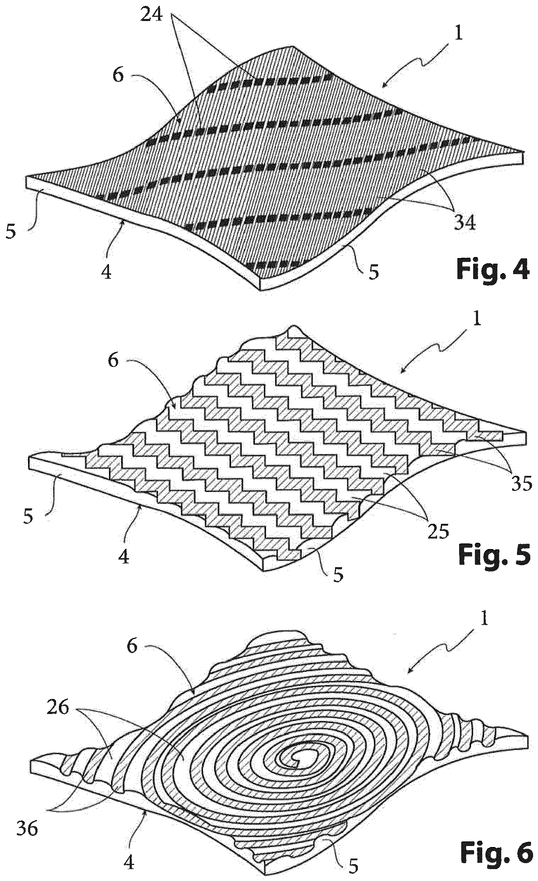

La figure 4 représente un textile (1) selon l'invention, ayant une face destinée à être en contact avec la peau (6) présentant des reliefs (24) en forme de nids-d'abeilles, espacés par des zones sans relief (34) et ayant une face sans relief (4). De tels reliefs (24) en forme de nids-d'abeilles permettent un pétrissage de la peau.

La figure 5 représente le textile (1) selon l'invention, ayant une face destinée à être au contact avec la peau (6) présentant des reliefs (25) en forme de vagues et une zone sans relief (35) et ayant une face sans relief (4). De tels reliefs (25) en forme de vagues permettent un effet veinotonique (activation du flux sanguin) et reproduisent les effets d'un massage palper-rouler.

La figure 6 représente le textile (1) selon l'invention, ayant une face destinée à être au contact avec la peau (6) présentant des reliefs (26) en forme de spirale, espacés par des zones sans relief (36) et ayant une face sans relief (4). De tels reliefs (26) en forme de spirale ont une action rayonnante et peuvent être utilisés de manière locale sur des articulations.

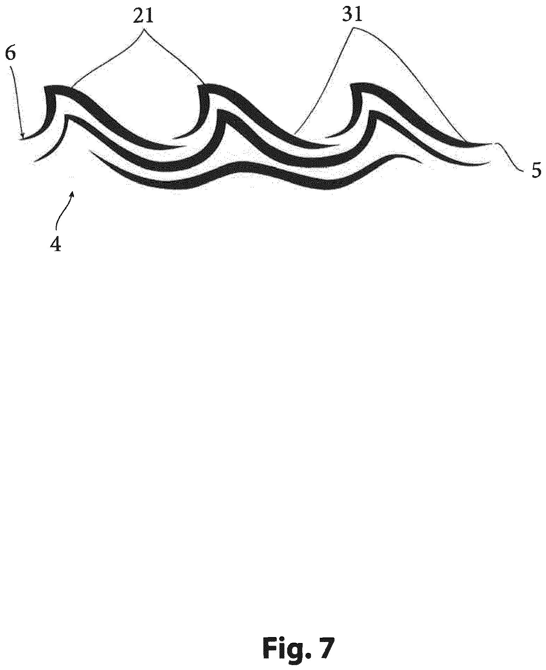

La figure 7 représente une vue en coupe (5) du textile (1) selon l'invention de la figure 1. La face sans relief (4) a une épaisseur supérieure ou égale à 1 mm. Les reliefs (21) de la face destinée à être au contact avec la peau (6) présentent une épaisseur supérieure ou égale à 3 mm par rapport aux zones (31) sans relief de ladite face (6).

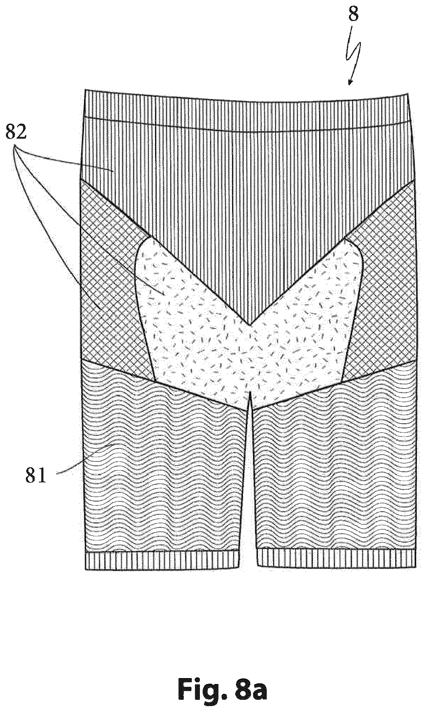

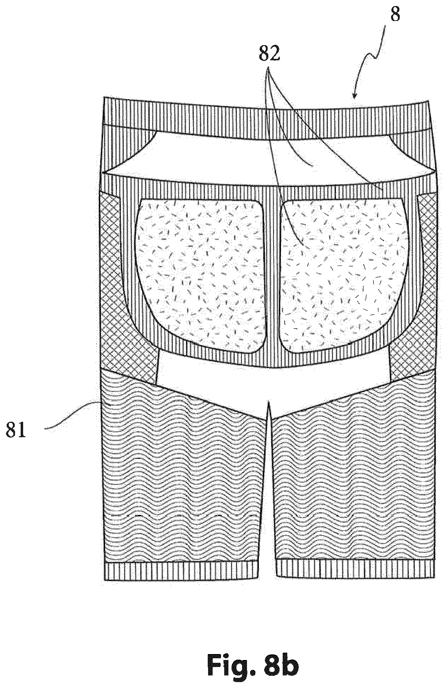

La figure 8 représente un shorty/corsaire ou legging (8) selon l'invention, face avant (fig. 8a) et face arrière (fig. 8b). Les zones (81) présentent des reliefs et les zones (82) n'ont pas de relief.

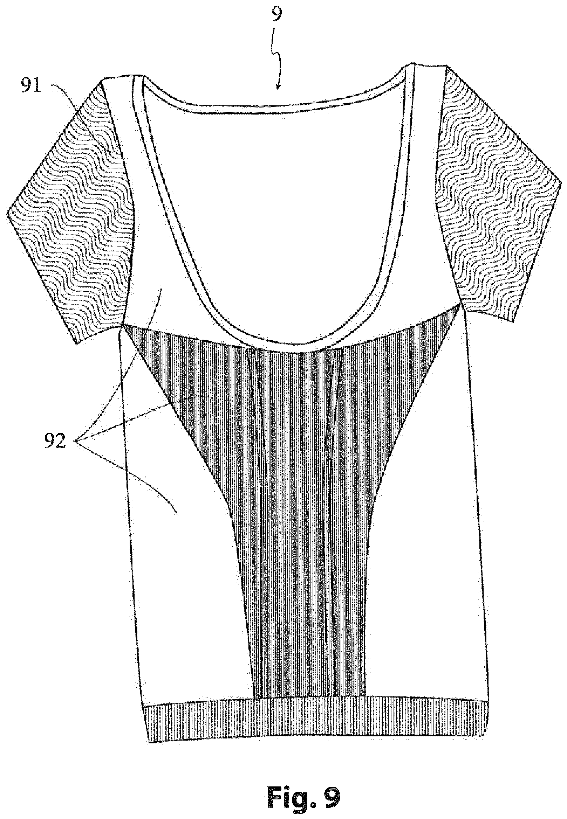

La figure 9 représente un maillot de corps (9) selon l'invention. Les zones (91) présentent des reliefs et les zones (92) n'ont pas de relief.

Le textile selon l'invention comprend au moins un relief qui est dessiné en fonction de la zone anatomique sur laquelle il est destiné à être appliqué.

Dans un mode de réalisation, le relief de la face destiné à être au contact de la peau (6) présente une épaisseur par rapport au niveau du textile sans relief supérieure ou égale à 3 mm.

Dans un mode de réalisation l'épaisseur totale du textile (1) est supérieure ou égale à 4 mm.

La norme ISO 5084:1996 prescrit une méthode pour déterminer l'épaisseur des textiles et produits textiles. Le mesureur d'épaisseur permet de mesurer la distance perpendiculairement entre deux plaques de référence appliquant sur l'échantillon une pression connue. L'épaisseur du textile peut par exemple être mesurée avec des contrôleurs d'épaisseur manuel ou palpeur mécanique manuel. Les Laboratoires VVC, entre autres commercialisent une gamme de mesureurs d'épaisseur de matériaux souples notamment du textile (compatibles avec la norme EN ISO 5084). Des exemples non limitatifs de testeurs multinormes sont commercialisés sous le nom VVC2000, VVC2005, VVC2010 ou VVC2020.

Le ou les reliefs sont obtenu(s) par le tissage ou le tricotage qui permettent de conférer à celui-ci une forme tridimensionnelle.

Dans un mode de réalisation, le textile (1) selon l'invention est caractérisé en ce que la forme du relief est choisie parmi des motifs tels que vagues, nids-d'abeilles, picots, spirales, lignes verticales et/ou horizontales.

Quand la forme du relief est choisie parmi les motifs tels que :
- vagues, on observe un effet veinotonique, activation du flux sanguin et imitation de massage palper-rouler,
- nids-d'abeilles, on observe un effet de pétrissage,
- picots, on observe une simulation de points d'acupuncture, traitement de contractions musculaires,
- spirales, on observe une action rayonnante sur les articulations.

Par exemple lorsqu'il s'agit de faire pénétrer un actif anti-cellulite et de masser une zone comprenant de la cellulite, les reliefs du textile vont être dessinés pour pouvoir reproduire un massage cutané du type palper-rouler et par exemple sous forme de vagues avec un relief supérieur à 4 mm.

Lorsque l'article textile est destiné à favoriser la microcirculation périphérique sur les jambes, les reliefs vont être des stries dont le relief sera inférieur à 4 mm et destinées à favoriser la pénétration de l'actif pharmaceutique.

Pour favoriser la pénétration par exemple d'un gel anti-inflammatoire sur une articulation le relief formera une spirale par exemple pour une application sur une articulation, par exemple une articulation du genou.

La présente invention concerne également l'utilisation d'un textile selon l'invention pour améliorer la pénétration de principes actifs ou de produits cosmétiques dans la peau.

L'invention concerne également un kit comprenant :
- une composition cosmétique et/ou des microcapsules contenant un ou plusieurs principes actifs ;
- un textile (1) selon l'invention.

L'invention concerne également une méthode de massage, caractérisée en ce qu'elle comprend l'application sur la peau d'un textile selon l'invention

Elle concerne également une méthode d'augmentation de l'efficacité de principes actifs, caractérisée en ce qu'elle comprend l'application sur la peau d'un textile (1) selon l'invention et d'au moins un principe actif.

La présente invention a également pour objet l'utilisation d'un textile (1) selon l'invention, pour la confection de vêtements, de préférence sans couture.

La présente invention a également pour objet un vêtement qui est constitué totalement ou partiellement du textile (1) tel que décrit ci-dessus. Le vêtement peut être choisi dans le groupe constitué par les gants, les chaussants, les sous-vêtements, les bas, les collants, les corsaires, les leggings, les maillots de corps.

La présente invention a aussi pour objet un article médical qui est constitué totalement ou partiellement du textile (1) tel que décrit ci-dessus. Cet article médical peut être choisi dans le groupe constitué par les bandages, les attelles, les pansements, les compresses, les bandeaux, les masques, les patches.

L'invention est davantage illustrée, mais de manière non limitative, par les exemples suivants.

### Exemple 1 : Résultats sur la minceur taille/hanches/cuisses.

Une étude démontre l'efficacité d'un corsaire formé par le textile selon l'invention. Le corsaire comprend des microcapsules de fucus. Le fucus une algue brune marine : *Fucus vesiculosus.* L'extrait de fucus est reconnu pour son action liporéductrice, anti-inflammatoire et antioxydante.

Des relevés centimétriques (taille, hanches et cuisses) ont été effectués à 0, 7 et 28 jours (J0, J7 et J28).

Un panel de 89 femmes âgés de plus de 18 ans (indice de masse corporelle (IMC) : de 23 à 27, taille de 1,55 m à 1,70 m) ayant un manque de fermeté au niveau des cuisses et de la cellulite ont été incluses dans cette étude sur une durée de 28 jours. Le corsaire a été porté 8h par jour.
Une mesure centimétrique du périmètre de la taille, des hanches et des cuisses a été effectuée afin d'évaluer l'efficacité amincissante. Les moyennes de mesure ainsi que les résultats maximum observés correspondant à la plus grande perte centimétrique (après 7 et 28 jours de test) et la perte moyenne du tiers supérieur du panel (correspondant aux 30 sujets observant les plus fortes pertes centimétriques) sont présentés dans les tableaux 1 et 2 ci-après.

Les résultats suivants ont été obtenus.
On observe une nette amélioration des valeurs centimétriques de façon homogène sur les trois points de mesure :

**Tableau 1 : résultats mesurés à J7.**

| 7 jours | | | |
|---|---|---|---|
| TAILLE | | | |
| Jour n° | Moyenne sur le tiers supérieur (cm) | Moyenne ensemble du panel (cm) | Maximum (cm) |
| J0 | **84.1** | 83.3 | |
| J7 | **81.6** | 82.4 | |
| **Différence J0/J7** | **-2.5** | **-0.9** | **-8** |

| HANCHES | | | |
|---|---|---|---|
| Jour n° | Moyenne sur le tiers supérieur (cm) | Moyenne ensemble du panel (cm) | Maximum (cm) |
| J0 | **98.3** | 98 | |
| J7 | **96** | 97.2 | |
| **Différence J0/J7** | **-2.3** | **-0.8** | **-16** |

| CUISSES | | | |
|---|---|---|---|
| Jour n° | Moyenne sur le tiers supérieur (cm) | Moyenne ensemble du panel (cm) | Maximum (cm) |
| J0 | **57.8** | 58.7 | |
| J7 | **56** | 57.9 | |
| **Différence J0/J7** | **-1.8** | **-0.8** | **-4,5** |

**Tableau 2 : Résultats mesurés à J28.**

| 28 jours | | | |
|---|---|---|---|
| TAILLE | | | |
| Jour n° | Moyenne sur le tiers supérieur (cm) | Moyenne ensemble du panel (cm) | Maximum (cm) |
| J0 | **84.9** | 83.4 | |
| J28 | **79.5** | 81.2 | |
| **Différence J0/J28** | **-5.4** | **-2.2** | **-12** |

| HANCHES | | | |
|---|---|---|---|
| Jour n° | Moyenne sur le tiers supérieur (cm) | Moyenne ensemble du panel (cm) | Maximum (cm) |
| J0 | **98.3** | 98 | |
| J28 | **93.9** | 96.1 | |
| **Différence J0/J28** | **-4.4** | **-1.9** | **-19** |

| CUISSES | | | |
|---|---|---|---|
| Jour n° | Moyenne sur le tiers supérieur (cm) | Moyenne ensemble du panel (cm) | Maximum (cm) |
| J0 | **58.8** | 58.7 | |
| J28 | **55.2** | 56.9 | |
| **Différence J0/J28** | **-3.7** | **-1.7** | **-7** |

Les pertes centimétriques sont statistiquement significatives après 7 et 28 jours de tests pour chaque localisation de mesure (taille, hanches et cuisses).

### Exemple 2 : Essais comparatifs

Les résultats de l'exemple 1 ont été comparés à ceux obtenus avec un textile constitué en partie de fibres céramiques, mais n'ayant pas de relief sur la surface destinée à être en contact avec la peau.

Les résultats suivants ont été obtenus sur 2 études réalisées sur les hanches et les cuisses et réalisées sur une période de 28 jours d'utilisation quotidienne, 8 heures par jour, sur un panel de 89 sujets.

Les résultats après 28 jours d'utilisation quotidienne, 8 heures par jour sont présentés dans le tableau 3.

**Tableau 3 : Résultats comparatifs hanches et cuisses à J28.**

| | | **Test réalisé sur un cosmétotextile constitué en partie de fibre céramique** | **Test réalisé sur un cosmétotextile constitué en partie de fibre céramique et texturé** |
|---|---|---|---|
| **HANCHES** | **max** | **4*** | **19*** |
| | **1/3 du panel** | **1,78** | **4,4** |
| **CUISSES** | **max** | **2*** | **7*** |
| | **1/3 du panel** | **0,5** | **3,7** |

| | | | |
|---|---|---|---|
| *Valeurs maximales mesurées. | | | |

Le tableau 3 indique que les valeurs maximales de perte centimétrique, tant au niveau des cuisses que des hanches sont toujours significativement supérieures avec le textile selon l'invention, de même que la moyenne du tiers supérieur du panel de sujets testés.

Les pertes centimétriques sont statistiquement significatives après 7 et 28 jours de test pour chaque localisation de mesure (hanches et cuisses).

### Exemple 3 : Mesures de températures cutanées avec et sans textile selon l'invention.

Sont reproduites ci-dessous les valeurs moyennes de montée en température mesurées entre un témoin sans textile (T1), un sujet porteur de textile incorporant un produit cosmétique (T2) et un sujet porteur de textile selon l'invention (T3). Les résultats présentés sont un moyenne des mesures sur un total de 7 mesures sur une journée :

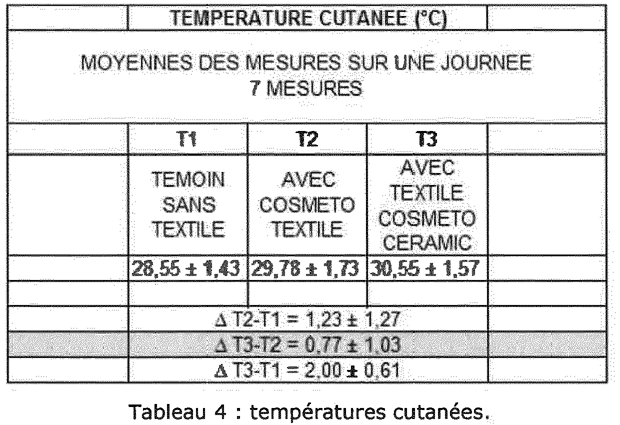

On observe un augmentation de température dûe au port du textile T2-T1 = 1,23°C et une augmentation de température : port du textile cosmeto ceramic T3-T2= 0.77°C (soit en moyenne 2,52% d'augmentation de température cutanée).

## Revendications

1. Textile (1), destiné à être appliqué sur la peau, constitué de fibres, fils ou filaments tissés ou tricotés comprenant :
- au moins une fibre, fil et/ou filament ayant la capacité d'émettre des radiations dans des longueurs d'ondes infrarouge lointain, de préférence entre 4 µm et 1000 µm, ou encore entre 5 µm et 20 µm,
**caractérisé en ce que** au moins une partie d'une face destinée à être en contact avec la peau (6) présente au moins un relief (21, 22, 23, 24, 25, 26).

2. Textile (1) selon la revendication 1, **caractérisé en ce que** le au moins une fibre, fil et/ou filament ayant la capacité d'émettre des radiations représente 10 à 50 % en masse des fibres, fils et/ou filaments du textile.

3. Textile (1) selon la revendication 1, **caractérisé en ce que** le au moins une fibre, fil et/ou filament ayant la capacité d'émettre des radiations est localisé dans le relief (21, 22, 23, 24, 25, 26).

4. Textile (1) selon la revendication 1, **caractérisé en ce que** le au moins une fibre fil et/ou filament est constitué d'au moins un polymère ou mélange de polymères.

5. Textile (1) selon la revendication 4, **caractérisé en ce que** le au moins une fibre fil et/ou filament est constitué d'au moins 50% de polyamide.

6. Textile (1) selon la revendication précédente, le polyamide est du polyamide 6.6.

7. Textile (1) selon l'une quelconque des revendications précédentes, **caractérisé en ce que** le relief (21, 22, 23, 24, 25, 26) de la face destiné à être au contact de la peau (6) présente une épaisseur par rapport au niveau du textile sans relief (31, 32, 33, 34, 35, 36) supérieure ou égale à 3 mm.

8. Textile (1) selon l'une quelconque des revendications précédentes, **caractérisé en ce que** l'épaisseur totale du textile est supérieure ou égale à 4 mm.

9. Textile (1) selon l'une quelconque des revendications précédentes, **caractérisé en ce que** la forme du relief (21, 22, 23, 24, 25, 26) est choisie parmi des motifs tels que vagues, nids-d'abeilles, picots, spirales, lignes verticales et/ou horizontales.

10. Textile (1) selon l'une quelconque des revendications précédentes, **caractérisé en ce que** la au moins une fibre, fil et/ou filament ayant la capacité d'émettre des radiations dans des longueurs d'ondes infrarouge lointain comprend au moins une charge minérale.

11. Textile (1) selon l'une quelconque des revendications précédentes, **caractérisé en ce que** la au moins une charge minérale sont des particules de céramique.

12. Textile (1) selon l'une quelconque des revendications précédentes, **caractérisé en ce qu'**il comprend en outre au moins un principe actif micro-encapsulé.

13. Kit comprenant :
- une composition cosmétique et/ou des microcapsules contenant un ou plusieurs principes actifs ;
- un textile (1) selon l'une des revendications 1 à 24.

14. Utilisation d'un textile selon la revendication 1 à 16 pour améliorer la pénétration de principes actifs ou de produits cosmétiques dans la peau.

15. Utilisation d'un textile (1) selon l'une quelconque des revendications 1 à 14 pour la confection de vêtements, de préférence sans couture.
